# EUROPEAN PATENT SPECIFICATION

(11) **EP 3 247 233 B1**
(45) Date of publication and mention of the grant of the patent: **15.05.2019**
(21) Application number: 16701857.1
(22) Date of filing: 21.01.2016
(51) Int. Cl.: A24F 47/00

(54) **VAPOUR PROVISION SYSTEM AND CARTRIDGE THEREFOR**
DAMPFBEREITSTELLUNGSSYSTEM UND PATRONE DAFÜR
SYSTÈME DE FOURNITURE DE VAPEUR ET CARTOUCHE CORRESPONDANTE

(30) Priority: 22.01.2015 GB 201501060
(43) Date of publication of application: 29.11.2017
(60) Divisional application: 19165920.0
(73) Proprietor: Nicoventures Holdings Limited, London WC2R 3LA (GB)
(72) Inventor: EWING, Mark Patrick Campbell, London WC2R 3LA (GB); SEAWARD, David Robert, London WC2R 3LA (GB); JEZEQUEL, Alexandre Julien, London WC2R 3LA (GB)
(74) Representative: D Young & Co LLP
(86) International application number: PCT/GB2016/050126
(87) International publication number: WO 2016/116754

(56) References cited:
- WO-A1-2012/126242
- WO-A1-2013/110208
- WO-A1-2014/207719

## Description

### Field

The present disclosure relates to a vapour provision system or device such as an electronic nicotine delivery system (e.g. an e-cigarette), and to a cartridge for use in such a device.

### Background

Electronic vapour provision systems such as e-cigarettes generally contain a cartridge to provide a reservoir of liquid which is to be vaporised, typically nicotine. When a user inhales on the device, a heater is activated to vaporise a small amount of liquid, which is therefore inhaled by the user. Once the reservoir of liquid has been exhausted, then at least a portion of the device containing the cartridge may be discarded to allow replacement with a new cartridge. Since the cartridge may therefore be a high-volume consumable, it is desirable that it can be produced in a cost-effective manner.
WO2013/110208A1 discloses an electronic simulation cigarette and a simulation cigarette mouthpiece comprising a cigarette mouthpiece sleeve made of a soft material which covers the outer wall of the simulation cigarette mouthpiece. However, this document does not teach, at least, an outer housing comprising a mechanical connector for longitudinally attaching a cartridge to a control unit of a vapour provision system.

### Summary

The disclosure is defined in the appended claims.

A cartridge is provided for use in a vapour provision system includes an inner container holding a reservoir of fluid to be vapourised, and an outer housing having a mouthpiece formed therein, wherein the outer housing extends in a longitudinal direction along the outside of the inner container for at least a substantial portion of the inner container. The inner container and outer housing are provided with a latch mechanism to retain the inner container within the outer housing. The outer housing comprises a mechanical connector for longitudinally attaching the cartridge to a control unit of a vapour provision system.

A vapour provision system that includes such a cartridge is also provided. This vapour provision system may be an electronic vapour provision device, such as an e-cigarette.

### Brief Description of the Drawings

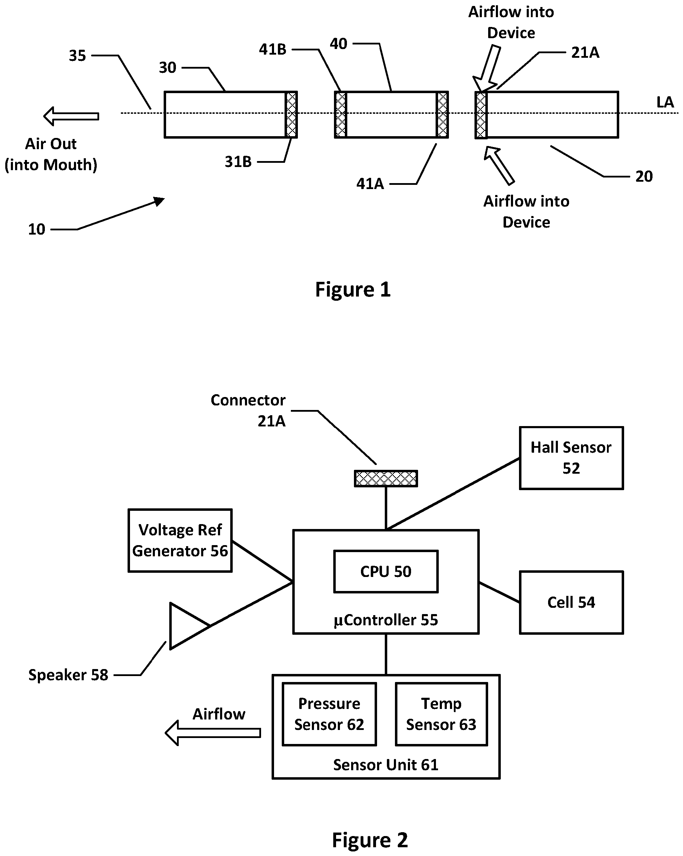

Figure 1 is a schematic (exploded) diagram of an e-cigarette in accordance with some embodiments of the disclosure.
Figure 2 is a schematic diagram of the main functional components of the body of the e-cigarette of Figure 1 in accordance with some embodiments of the disclosure.

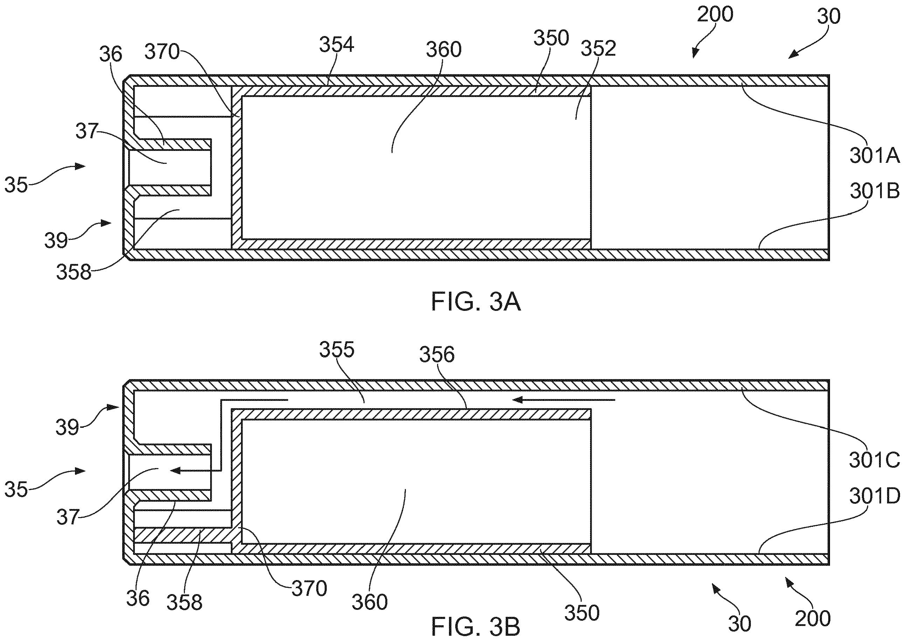

Figures 3A and 3B are schematic diagrams of the cartridge portion of an e-cigarette according to an existing design; in particular, Figures 3A and 3B are two sections taken in mutually orthogonal first and second planes that both include the longitudinal axis LA of the e-cigarette.

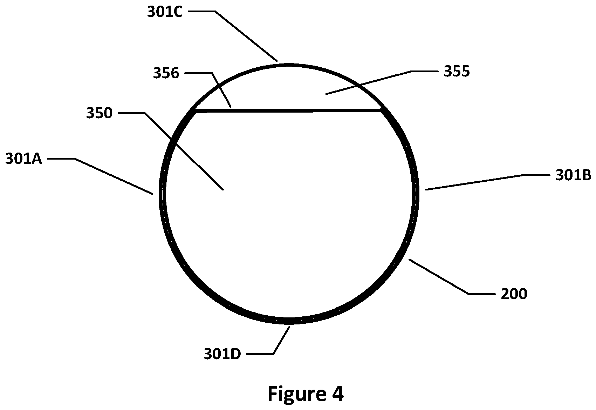

Figure 4 is schematic diagram of the cartridge portion of the e-cigarette of Figure 3 according to an existing design and shows a section through the cartridge portion in a plane perpendicular to the longitudinal axis LA, taken approximately halfway along the length of the cartridge portion.

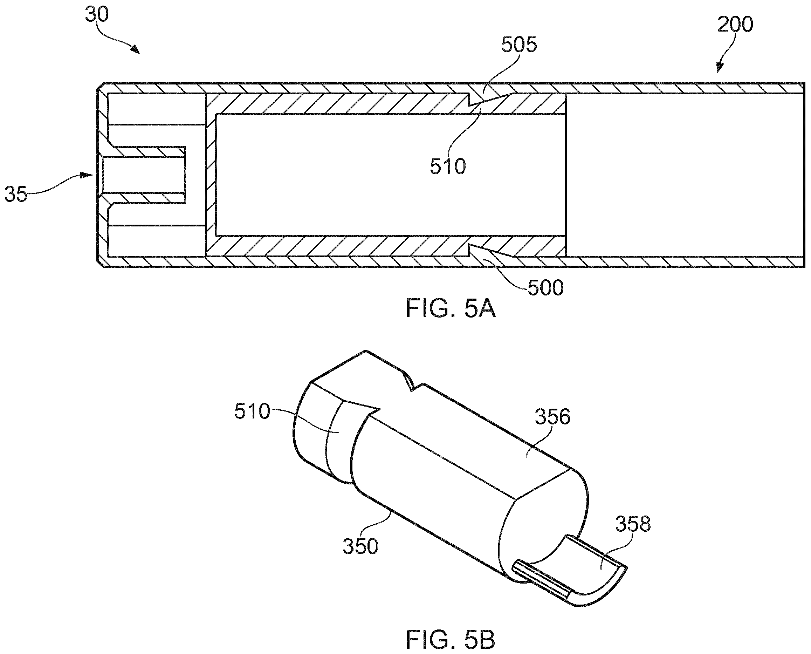

Figures 5A and 5B illustrate one implementation of the cartridge for an e-cigarette in accordance with some embodiments of the invention, where Figure 5A is a horizontal cross-section through the cartridge (including the longitudinal axis), while Figure 5B is a view of the inner container by itself (i.e. as removed from inside the outer housing).

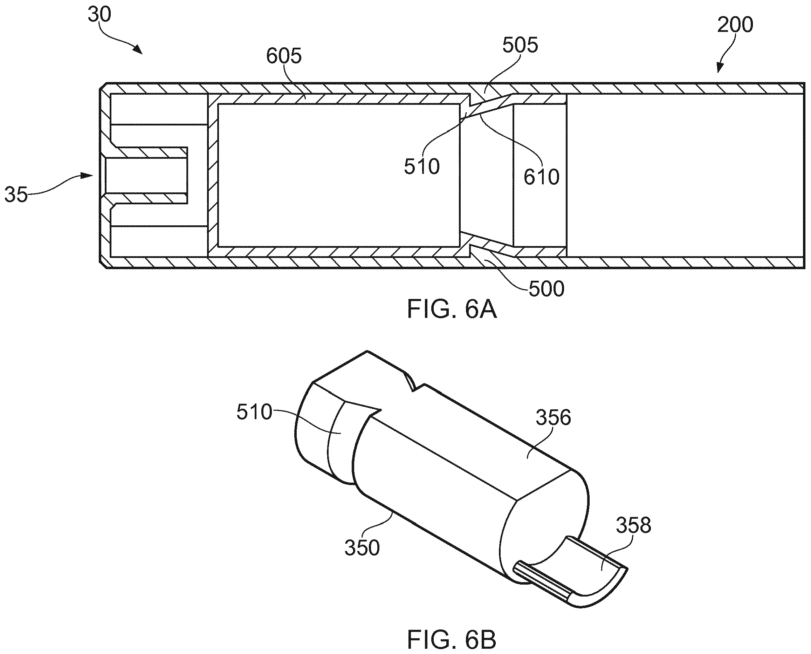

Figures 6A and 6B illustrate one implementation of the cartridge for an e-cigarette in accordance with some embodiments of the invention, where Figure 6A is a horizontal cross-section through the cartridge (including the longitudinal axis), while Figure 6B is a view of the inner container by itself (i.e. as removed from inside the outer housing).

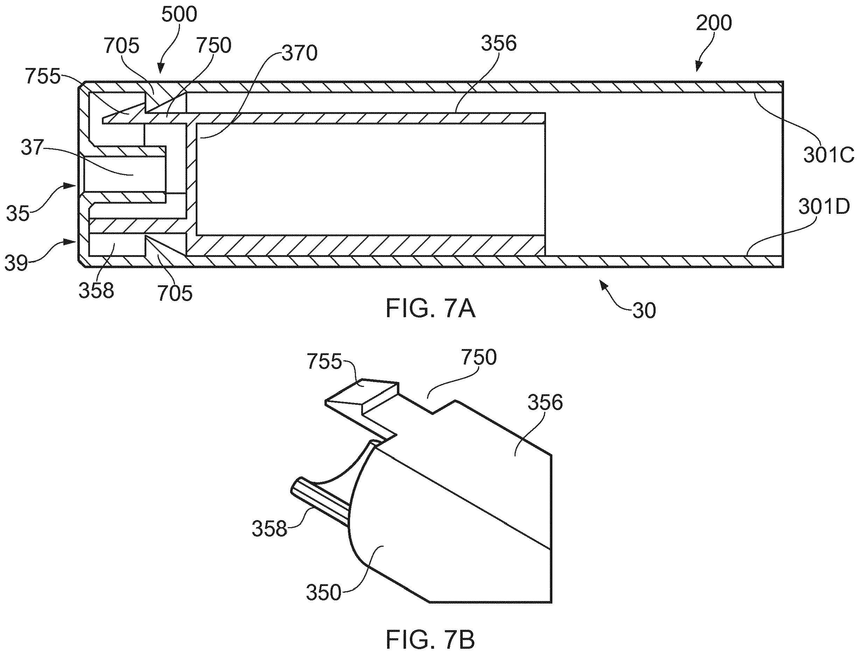

Figures 7A and 7B illustrate one implementation of the cartridge for an e-cigarette in accordance with some embodiments of the invention, where Figure 7A is a horizontal cross-section through the cartridge (including the longitudinal axis), while Figure 7B is a view of the mouth end portion of the inner container by itself (i.e. as removed from inside the outer housing).

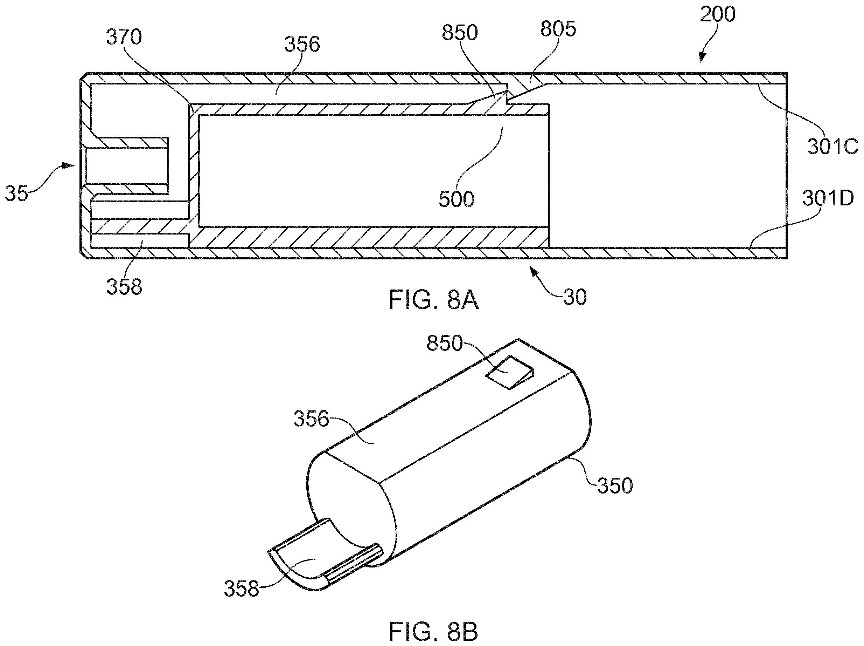

Figures 8A and 8B illustrate one implementation of the cartridge for an e-cigarette in accordance with some embodiments of the invention, where Figure 8A is a horizontal cross-section through the cartridge (including the longitudinal axis), while Figure 8B is a view of the inner container by itself (i.e. as removed from inside the outer housing).

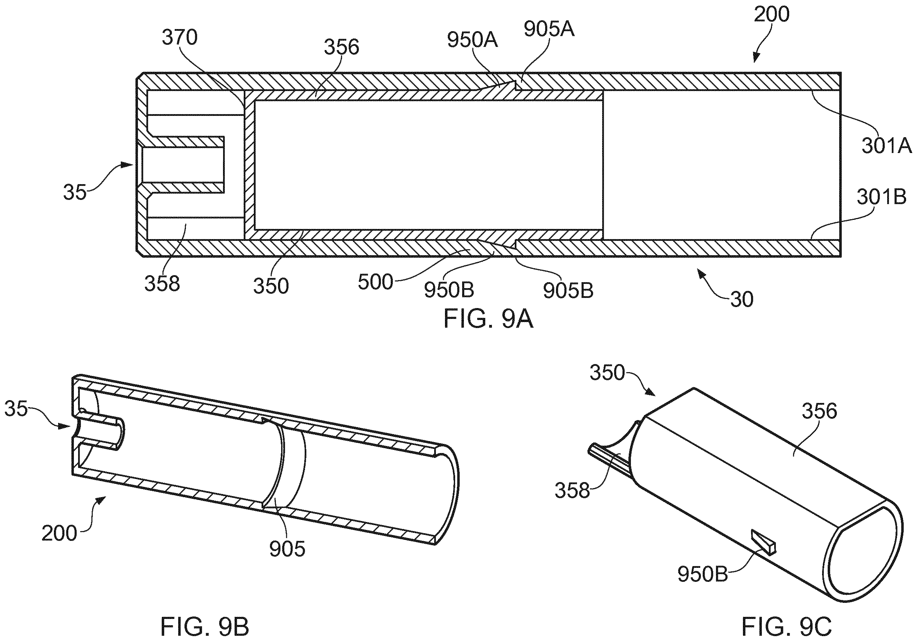

Figures 9A, 9B and 9CB illustrate one implementation of the cartridge for an e-cigarette in accordance with some embodiments of the invention, where Figure 9A is a horizontal cross-section through the cartridge (including the longitudinal axis), Figure 9B is a view of the outer housing (i.e. without the inner container) sectioned down a vertical plane (including the longitudinal axis), and Figure 9 is a view of the inner container by itself (i.e. as removed from inside the outer housing).

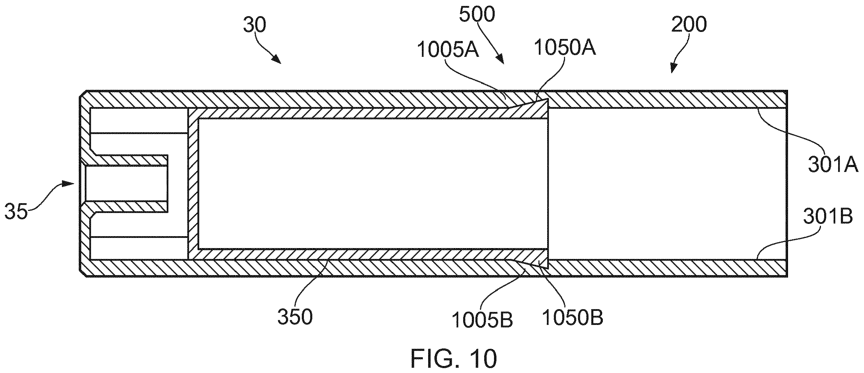

Figure 10 illustrate one implementation of the cartridge for an e-cigarette in accordance with some embodiments of the invention, showing a horizontal cross-section through the cartridge (including the longitudinal axis).

### Detailed Description

As described above, the present disclosure relates to an vapour provision system, such as an e-cigarette. Throughout the following description the term "e-cigarette" is used; however, this term may be used interchangeably with (electronic) vapour provision system.

Figure 1 is a schematic (exploded) diagram of an e-cigarette 10 in accordance with some embodiments of the disclosure (not to scale). The e-cigarette comprises a body (control unit) 20, a cartridge 30 and a vaporiser 40. The cartridge includes an internal chamber containing a reservoir of liquid and a mouthpiece 35. The liquid in the reservoir typically includes nicotine in an appropriate solvent, and may include further constituents, for example, to aid aerosol formation, and/or for additional flavouring. The cartridge reservoir may include a foam matrix or any other structure for retaining the liquid until such time that it is required to be delivered to the vaporiser. The control unit 20 includes a re-chargeable cell or battery to provide power to the e-cigarette 10 and a circuit board for generally controlling the e-cigarette. The vaporiser 40 includes a heater for vaporising the liquid and further includes a wick or similar device which transports a small amount of liquid from the reservoir in the cartridge to a heating location on or adjacent the heater. When the heater receives power from the battery, as controlled by the circuit board, the heater vaporises the liquid from the wick and this vapour is then inhaled by a user through the mouthpiece.

The control unit 20 and the vaporiser 40 are detachable from one another, but are joined together when the device 10 is in use, for example, by a screw or bayonet fitting (indicated schematically in Figure 1 as 41A and 21A). The connection between the control unit and the vaporiser provides for mechanical and electrical connectivity between the two. When the control unit is detached from the vaporiser, the electrical connection 21A on the control unit that is used to connect to the vaporiser also serves as a socket for connecting a charging device (not shown). The other end of the charging device can be plugged into a USB socket to re-charge the cell in the control unit of the e-cigarette. In other implementations, the e-cigarette may be provided with a cable for direction connection between the electrical connection 21A and a USB socket.

The control unit is provided with one or more holes (not shown in Figure 1) for air inlet. These holes connect to an air passage through the control unit to an air passage provided through the connector 21A. This then links to an air path through the vaporiser 40 and the cartridge 30 to the mouthpiece 35. The cartridge 30 and the vaporiser 40 are attached in use by connectors 41B and 31B (again shown schematically in Figure 1). As explained above, the cartridge includes a chamber containing a reservoir of liquid, and a mouthpiece. When a user inhales through the mouthpiece 35, air is drawn into the control unit 20 through one or more air inlet holes. This airflow (or the resulting change in pressure) is detected by a pressure sensor, which in turn activates the heater to vaporise the liquid from the cartridge. The airflow passes from the control unit, through the vaporiser, where it combines with the vapour, and this combination of airflow and (nicotine) vapour then passes through the cartridge and out of the mouthpiece 35 to be inhaled by a user. The cartridge 30 may be detached from the vaporiser 40 and disposed of when the supply of liquid is exhausted (and then replaced with another cartridge). Note that there is no facility for a user to re-fill the cartridge.

The e-cigarette 10 has a longitudinal or cylindrical axis which extends along the centre-line of the e-cigarette from the mouthpiece 35 at one end of the cartridge 30 to the opposing end of the control unit 20 (usually referred to as the tip end). This longitudinal axis is indicated in Figure 1 by the dashed line denoted LA.

It will be appreciated that the e-cigarette 10 shown in Figure 1 is presented by way of example, and various other implementations can be adopted. For example, in some embodiments, the vaporiser 40 may be integrated into the cartridge as a single unit (sometimes referred to as a cartomiser), and the charging facility may connect to an additional or alternative power source, such as a car cigarette lighter.

Figure 2 is a schematic diagram of the main functional components of the control unit 20 of the e-cigarette 10 of Figure 1 in accordance with some embodiments of the disclosure. These components may be mounted on the circuit board provided within the control unit 20, although depending on the particular configuration, in some embodiments, one or more of the components may instead be accommodated in the control unit to operate in conjunction with the circuit board, but are not physically mounted on the circuit board itself.

The control unit 20 includes a sensor unit 61 located in or adjacent to the air path through the control unit 20 from the air inlet to the air outlet (to the vaporiser). The sensor unit includes a pressure sensor 62 and temperature sensor 63 (also in or adjacent to this air path). The control unit further includes a Hall effect sensor 52, a voltage reference generator 56, a small speaker 58, and an electrical socket or connector 21A for connecting to the vaporiser 40 or to a USB charging device.

The microcontroller 55 includes a CPU 50. The operations of the CPU 50 and other electronic components, such as the pressure sensor 62, are generally controlled at least in part by software programs running on the CPU (or other component). Such software programs may be stored in non-volatile memory, such as ROM, which can be integrated into the microcontroller 55 itself, or provided as a separate component. The CPU may access the ROM to load and execute individual software programs as and when required. The microcontroller 55 also contains appropriate communications interfaces (and control software) for communicating as appropriate with other devices in the control unit 10, such as the pressure sensor 62.

The CPU controls the speaker 58 to produce audio output to reflect conditions or states within the e-cigarette, such as a low battery warning. Different signals for signalling different states or conditions may be provided by utilising tones or beeps of different pitch and/or duration, and/or by providing multiple such beeps or tones.

As noted above, the e-cigarette 10 provides an air path from the air inlet through the e-cigarette, past the pressure sensor 62 and the heater (in the vaporiser), to the mouthpiece 35. Thus when a user inhales on the mouthpiece of the e-cigarette, the CPU 50 detects such inhalation based on information from the pressure sensor. In response to such a detection, the CPU supplies power from the battery or cell 54 to the heater, which thereby heats and vaporises the liquid from the wick for inhalation by the user.

Figures 3A and 3B, plus Figure 4, are schematic diagrams of the cartridge portion 30 of e-cigarette 10 according to an existing design. Figure 4 shows a section through the cartridge portion in a plane perpendicular to the longitudinal axis LA, taken approximately halfway along the length of the cartridge portion. Figures 3A and 3B are two sections taken in first and second planes that both include the longitudinal axis LA. These first and second planes are orthogonal to another. For convenience, we will refer to the first plane shown in Figure 3A as a horizontal plane, and the second plane shown in Figure 3B as the vertical plane. However, it will be appreciated that although in normal use, the longitudinal axis LA of the e-cigarette 10 is approximately horizontal, a user may typically hold the e-cigarette at any rotational (azimuthal) angle around this longitudinal axis. Accordingly, the terms vertical and horizontal are adopted for ease of explanation, rather than particularly implying a given orientation of the device for use.

As shown in Figures 3A, 3B and 4, the cartridge contains two main portions: an outer housing 200 and an inner container 350. The outer housing 200 has a generally circular cross-section in a plane perpendicular to the longitudinal axis LA, as can be seen in Figure 4, thereby forming a generally cylindrical tube. The outer housing has opposing side walls 301A, 301B, plus opposing top and bottom walls 301C and 301D respectively. (It will be appreciated that these walls 301A-D are generally just different, circumferentially spaced, portions of the tube forming the outer housing 200).

One end of the outer housing tube, corresponding to the location of the mouthpiece 35, is partly closed by an end wall 39, which is perpendicular to the longitudinal axis LA. An aperture is formed in the centre of this end wall, and in particular, an inner tube 37 is formed, which is defined by inner wall 36. This inner wall 36 likewise forms a generally cylindrical tube, parallel to the main outer tube of the outer housing 200 formed by walls 301A-D. However, this inner tube only extends inwards (along the longitudinal axis LA) a relatively short distance from the radially innermost portion of end wall 39 (compared with the length of the outer tube).

The inner container 350 also has a generally circular cross-section in a plane perpendicular to the longitudinal axis LA, thereby forming a generally cylindrical tube. In particular, the inner container thereby defines a central cavity 360 which retains a reservoir of liquid which is to be vaporised, typically nicotine (in solution). The opening 352 of the inner container at the end opposite to the mouthpiece, as shown in Figure 3A, may be closed with a thin wall, e.g. using metallic foil, to create the sealed chamber. The liquid may be held inside the sealed chamber in a foam matrix. The interior surface of the outer housing 200 may include a screw thread at the end opposite to the mouth end 35 to join to attach the cartridge 30 to the vaporiser portion 40 (see Figure 1). The attachment may cause a wick on the vaporiser portion to penetrate the cartridge (e.g. by puncturing the seal on the reservoir), thereby drawing liquid from the reservoir onto the vaporiser. (Please note that details of the end of the outer housing 200 and the container 350 which are furthest from the mouthpiece 35, including the thin wall or other seal, and the configuration of the wick, etc, are omitted for clarity from Figures 3A and 3B).

The horizontal side walls of the inner container 350 abut against the corresponding side walls 301A, 301 B of the outer housing. In particular, there is an interference fit between the horizontal side walls of the inner container 350 and the corresponding side walls 301A, 301B of the outer housing, which is used to retain the inner container 350 within the outer housing 200. A portion of this interference fit is denoted by reference numeral 354 in Figure 3A, and is formed between the side wall 301A of the outer housing 200 and the corresponding side wall of the inner container. Note that in practice there is a slight taper on the outer housing 200 (not shown in Figure 3) in order to enable moulding and to support this interference fit - i.e. the outer housing tapers slightly inwards so as to be narrower at the mouth end.

The generally cylindrical tube of the inner container 350 is closed at the mouthpiece end by wall 370. In addition, the interference fit between the side wall 301A of the outer housing 200 and the corresponding side wall of the inner container generally prevents the flow of air along the e-cigarette 10. Accordingly, although the inner container 350 has a generally circular cross-section in a plane perpendicular to the longitudinal axis LA, the topmost portion of this circle is flattened to allow airflow through the e-cigarette 10.

In particular, the top wall 356 of the inner container 350 is formed (in the cross-section of Figure 4) by a chord, rather than by an arc. This therefore defines an air passage 355 between the top wall 301C of the outer housing 200 and the top wall 356 of the inner container 350. This air passage 355 is also shown in Figure 3B, together with arrows denoting the airflow from the vaporiser portion 40 out through the mouthpiece 35.

The end wall 370 of the inner container 350 which is adjacent the mouthpiece 35 is provided with a tab 358. This tab extends in a direction parallel to the longitudinal axis LA of the e-cigarette 10 to abut against the end wall 39 of the outer housing 200. The tab has a cross-section of an arc in a plane perpendicular to the longitudinal axis LA of the e-cigarette 10, and is located at the bottom of the inner container 350, i.e. opposite to the top wall 356. In this position, the tab 358 does not block the airflow from the passage 355 out through the mouthpiece 35.

In addition, the length of the tab 358 (in a direction parallel to the longitudinal axis LA) is greater than the length of the inner wall 36 which defines the mouthpiece tube 37. Consequently, the tab 358 serves to prevent the end wall 370 abutting against (and thereby closing) the inside end of the mouthpiece tube 37. This configuration therefore again helps to ensure that air flowing through the air passage 355 can then reach the mouthpiece tube 37 in order to exit through the mouthpiece 35.

While the cartridge 30 according to the existing design, as shown in Figures 3A, 3B and 4, is functional, this design places strict tolerances on the relative sizing of the inner container 350 relative to the outer housing 200 in order to ensure that the interference fit 354 can be successfully achieved. Thus if the outer housing 200 is too large relative to the inner container 350, the inner container may become dislodged from its correct positioning in the cartridge. Conversely, if the outer housing 200 is too small relative to the inner container 350, then it may not be possible to insert the inner container into the outer housing 200. The strict tolerances on the relative sizing of the inner container 350 relative to the outer housing 200 can increase manufacturing costs and/or cause product reliability issues.

In order to address the above concerns, a cartridge 30 has been developed in which the inner container 350 and outer housing 200 are latched together by a resilient latching mechanism. It will be appreciated that at least one of the inner container 350 and/or outer housing 200 is usually made of plastic, which typically provides sufficient flexibility or resilience to support such a latching mechanism.

Figures 5-10 illustrate various different implementations of the latching mechanism. These implementations can be considered as modifications of the cartridge 30 described with respect to Figures 3 and 4. Thus in the discussion of Figures 5-10, aspects of these implementations which are generally unchanged from the cartridge 30 already described with respect to Figures 3 and 4 will not be described again in order to avoid repetition. Furthermore, it will be appreciated that the various implementations of Figures 5-10 are not intended to be exhaustive - rather the skilled person will be aware of various possible further implementations. In addition, the various implementations of Figures 5-10 are not intended to be mutually exclusive, in that one or more features from different implementations may be combined as appropriate to create new implementations.

Figures 5A and 5B illustrate one implementation of the cartridge 30 which again comprises an outer housing 200 and an inner container 350. In particular, Figure 5A is a horizontal cross-section through the cartridge 30 (including the longitudinal axis LA), while Figure 5B is a view of the inner container 350 by itself (i.e. as removed from inside the outer housing 200).

The implementation of Figures 5A and 5B differs from the cartridge of Figures 3A, 3B and 4 by the inclusion of a latching mechanism 500. This latching mechanism is formed by the provision of a groove 510 formed in the inner container 350 and a corresponding protrusion 505 formed on the inside of the outer housing 200. As can be seen in Figure 5B, the groove 510 extends around the circumference of the inner container (with respect to the longitudinal axis), except that it does not extend across the top wall 356. The groove has a shape somewhat analogous to the numeral "7" and is formed by two sides. The first side is located furthest from the mouthpiece 35 and has a relative shallow angle or gradient with respect to the longitudinal axis LA, and with respect to the external cylindrical surface of the inner container 350. The second side is located closer to the mouthpiece 35 and has a much steeper (potentially perpendicular) angle or gradient with respect to the longitudinal axis LA.

The protrusion 505 formed on the inside of the outer housing 200 has a complementary shape to the groove 510. In particular, the protrusion 505 extends around the circumference of the inner wall of the outer housing 200. However, the protrusion does not extend across the top wall 301C of the outer housing 200 in order not to obstruct the air passage 355. The protrusion 505 also has a shape somewhat analogous to the numeral "7" (in order to match the groove 510) and is formed by two sides. The first side is located furthest from the mouthpiece 35 and has a relative shallow angle or gradient with respect to the longitudinal axis LA, and with respect to the internal cylindrical surface of the outer housing 200. The second side is located closer to the mouthpiece 35 and has a much steeper (potentially perpendicular) angle or gradient with respect to the longitudinal axis LA.

It will be appreciated that once the inner container 350 has been inserted into the outer housing 200 as per the implementation shown in Figure 5, the steep second side of the protrusion 505 abuts against the steep second side of the groove 510. This abutment prevents movement between the inner container 350 and the outer housing 200 along the longitudinal axis, especially in a direction that would tend to move the inner container towards the end of the outer housing 200 furthest from the mouthpiece 35.

Figures 6A and 6B illustrate another implementation of the cartridge 30 which again comprises an outer housing 200 and an inner container 350. In particular, Figure 6A is a horizontal cross-section through the cartridge 30 (including the longitudinal axis LA), while Figure 6B is a view of the inner container 350 by itself (i.e. as removed from inside the outer housing 200).

The implementation of Figures 6A and 6B is similar to the implementation of Figures 5A and 5B. The difference is that in the implementation of Figures 5A and 5B, the internal face of wall of the inner container 350 was flat along the longitudinal length of the cartridge. Consequently, the portion of the wall of the inner container 350 where the groove 510 is formed is thinner, and hence potentially weaker, than the remainder of this wall. In the implementation of Figures 6A and 6B however, the wall 605 of the inner container in effect has an approximately constant thickness. This means that the indentation of the groove 510 is mirrored by corresponding indentation 610 of the wall 605 of the inner container into the internal volume of the inner container.

It will be appreciated that the operation of the latching mechanism 500 in the implementation of Figures 6A and 6B is substantially similar to the operation of the latching mechanism 500 in the implementation of Figures 5A and 5B. However, the implementation of Figures 6A and 6B avoids having a reduced thickness for the wall 605 of the inner container 350, which may be important for some situations.

Figures 7A and 7B illustrate another implementation of the cartridge 30 which again comprises an outer housing 200 and an inner container 350. In particular, Figure 7A is a vertical cross-section through the cartridge 30 (including the longitudinal axis LA), while Figure 7B is a view of the inner container 350 by itself (i.e. as removed from inside the outer housing 200), in particular the portion adjacent the mouth end 35.

In the implementation of Figures 7A and 7B, the outer housing 200 is generally the same as for the implementation of Figures 3A, 3B and 4, except for the addition of a protrusion 705. This protrusion is located near the mouthpiece end of the outer housing 200, in particular, between the end wall 370 of the inner container and the end wall 39 of the outer housing. The protrusion 705 is directly radially inward and is formed all around the inner circumference of the outer housing 200, i.e. it spans azimuth angles form 0 to 360 degrees with respect to the longitudinal axis LA.

The protrusion 705 again has a shape somewhat analogous to the numeral "7", and is formed by two sides. The first side (a ramp portion) is located furthest from the mouthpiece end 35 and has a relative shallow angle or gradient with respect to the longitudinal axis LA, and also with respect to the internal cylindrical surface of the outer housing 200. The second side (a catch portion) is located closer to the mouthpiece 35 and has a much steeper (potentially perpendicular) angle or gradient with respect to the longitudinal axis LA.

In the implementation of Figures 7A and 7B, the inner container 350 is generally the same as for the implementation of Figures 3A, 3B and 4, except for the addition of a second tab 750. This second tab 750 is like the first tab, in that it extends from wall 370 towards the mouthpiece end 35. However, the second tab is somewhat shorter than the first tab, so that it does not reach the end wall 39 of the outer housing 200. In addition, the second tab 750 extends from the top wall 356 of the inner container, and is therefore diametrically opposed (having regard to the longitudinal axis LA) to the first tab 358, which extends from close to the bottom of the inner container 350.

The second tab 750 is also shaped differently from the first tab 358. Thus the second tab 750 comprises a first portion, which is flat and attached to the end wall 370. This flat portion can in effect be considered as an extension of the top wall 356. The flat portion also supports, in cantilever fashion a raised portion 755. This raised portion 755 interacts with the protrusion 705 of the outer housing 200 to form the latching mechanism 500. Note however that the protrusion 705 of the outer housing is sized so as not to obstruct the first tab 358, which can pass radially inward of the protrusion 705.

The raised portion 755 again has a shape somewhat analogous to the numeral "7", and is formed by two sides. The first side is located furthest from the end wall 370 and has a relative shallow angle or gradient with respect to the longitudinal axis LA, and also with respect to the top wall 356 of the inner container 350. The second side is located closer to the mouthpiece 35 and has a much steeper (potentially perpendicular) angle or gradient with respect to the longitudinal axis LA.

It can be seen that in operation, as the inner container is inserted into the outer housing 200, the raised portion 755 of the second tab 750 makes contact with the inward protrusion 705 of the outer container. This causes the second tab 750 to flex slightly in a radially inward (downward) direction, thereby allowing the raised portion 755 to slide past (and against) the inward protrusion 705.

Eventually, when the inner container is fully inserted, as shown in Figure 7A, the corner of the protrusion 705 (i.e. where the shallow side meets the steep side) goes past the corner of the raised portion 755 (again where the shallow side meets the steep side). This allows the second tab 750 to flex resiliently back upwards to the position shown in Figure 7A. In this configuration, the steep side of the protrusion 705, which faces in the direction of the mouth end 35, abuts against the steep side of the raised portion 755 of the second tab 750, which faces in the opposite direction (away from the mouth end). These two sides abut against one another to provide a latching action for latch mechanism 500, and thereby prevent withdrawal of the inner container 350 from the outer housing 200.

Note that the circumferential (azimuthal) extent, i.e. the rotational angle subtended with respect to the longitudinal axis LA, is smaller for the second tab 750 than for the first tab 358. In addition, the rotational angle subtended with respect to the longitudinal axis LA, is smaller for the second tab 750 than for the top wall 356 of the inner container. This ensures that air flowing along the passage 355 (see Figure 3B) is able to flow around the second tab, i.e. on either side of it, in order to progress to the mouthpiece hole 37 and then out of the e-cigarette 10.

One particular advantage of the implementation shown in Figures 7A and 7B is that for inserting the inner container 350 into the outer housing 200, the two longitudinal axes for these two components must be mutually aligned (i.e. coincident). However, there is no need to rotationally align the inner container 350 relative to the outer housing 200 about the longitudinal axis LA, since the inward protrusion 705 of the outer housing spans a rotational angle of 360 degrees. Accordingly, the second tab 750 will engage with the inward protrusion irrespective of the relative rotational angle of insertion between the outer housing 200 and the inner container 350. This therefore avoids the need to perform a rotational alignment between these two components prior to insertion of the inner container 350 into the outer housing 200, which can help to reduce manufacturing complexity (and hence costs).

The embodiment of Figures 7A and 7B again avoids having a groove portion formed in the inner container 350, thereby avoiding any potential weakness. In addition, unlike the embodiment of Figure 6, the internal shape of the inner container is unchanged. This may help to retain the maximum volume of the inner container 350, as well as avoiding any potential difficulties with the filling process.

Figures 8A and 8B illustrate another implementation of the cartridge 30 which again comprises an outer housing 200 and an inner container 350. In particular, Figure 8A is a verticall cross-section through the cartridge 30 (including the longitudinal axis LA), while Figure 8B is a view of the inner container 350 by itself (i.e. as removed from inside the outer housing 200).

In the implementation of Figures 8A and 8B, the outer housing 200 is generally the same as for the implementation of Figures 3A, 3B and 4, except for the addition of a protrusion 805. This protrusion is generally similar to the protrusion 705 in the implementation of Figures 7A and 7B, except for its location. Thus the protrusion 805 is not near the mouth end 35, but rather is located so as to be near the end of the inner container 350 furthest from the mouth end 35 (when the inner container has been fully inserted into the outer housing 200).

The protrusion 805 is directly radially inward and again has a shape somewhat analogous to the numeral "7", and is formed by two sides. The first side (a ramp portion) is located furthest from the mouthpiece end 35 and has a relative shallow angle or gradient with respect to the longitudinal axis LA, and also with respect to the internal cylindrical surface of the outer housing 200. The second side (a catch portion) is located closer to the mouthpiece 35 and has a much steeper (potentially perpendicular) angle or gradient with respect to the longitudinal axis LA.

In the implementation of Figures 8A and 8B, the inner container 350 is generally the same as for the implementation of Figures 3A, 3B and 4, except for the addition of a protrusion 850 formed on the top wall 356 of the inner container and directed radially outward. The protrusion 850 again has a shape somewhat analogous to the numeral "7", and is formed by two sides. The first side is located closest to the end wall 370 and has a relative shallow angle or gradient (ramp portion) with respect to the longitudinal axis LA, and also with respect to the top wall 356 of the inner container 350. The second side is located further from the end wall 370 and has a much steeper (potentially perpendicular) angle or gradient with respect to the longitudinal axis LA.

In operation, as the inner container is inserted into the outer housing 200, the ramp portion of the protrusion 850 on the inner container 350 makes contact with the corresponding ramp portion of the inward protrusion 805 of the outer container. Eventually, when the inner container is fully inserted, as shown in Figure 8A, the steep side of the protrusion 805, which faces in the direction of the mouth end 35, abuts against the steep side of the inner container protrusion 850, which faces in the opposite direction (away from the mouth end). These two sides abut against one another to provide a latching action for latch mechanism 500, and thereby prevent withdrawal of the inner container 350 from the outer housing 200.

Note that the width of the protrusion 850 on the top wall 356 of the inner container 350 is less than the width of the top wall 356. This ensures that air flowing along the passage 355 (see Figure 3B) is able to flow around the protrusion 850, i.e. on either side of it, in order to progress to the mouthpiece hole 37 and then out of the e-cigarette 10.

Figures 9A, 9B and 9C illustrate another implementation of the cartridge 30 which again comprises an outer housing 200 and an inner container 350. In particular, Figure 9A is a horizontal cross-section through the cartridge 30 (including the longitudinal axis LA), Figure 9B is a view of the outer housing 200 (without the inner container 350) which has been split down a vertical plane, and Figure 9C is a view of the inner container 350 by itself (i.e. as removed from inside the outer housing 200).

In the implementation of Figures 9A-9C, the outer housing 200 is generally the same as for the implementation of Figures 3A, 3B and 4, except for the addition of a circumferential groove 905 in the inner wall of the outer housing. More particularly, the groove 905 is formed all around the inner circumference of the outer housing 200, i.e. it spans azimuth angles form 0 to 360 degrees with respect to the longitudinal axis LA. The groove 905 again has a shape somewhat analogous to the numeral "7", and is formed by two sides. The first side (a ramp portion) is located furthest from the mouthpiece end 35 and has a relative shallow angle or gradient with respect to the longitudinal axis LA, and also with respect to the internal cylindrical surface of the outer housing 200. The second side (a catch portion) is located closer to the mouthpiece 35 and has a much steeper (potentially perpendicular) angle or gradient with respect to the longitudinal axis LA.

In the implementation of Figures 9A-9C, the inner container 350 is generally the same as for the implementation of Figures 3A, 3B and 4, except for the addition of a protrusion 950A, 950B formed on each side wall of the inner container and directed radially outward. The protrusions 950A, 950B again have a shape somewhat analogous to the numeral "7", and are each formed by two sides. The first side is located closest to the end wall 370 and has a relative shallow angle or gradient (ramp portion) with respect to the longitudinal axis LA. The second side is located further from the end wall 370 and has a much steeper (potentially perpendicular) angle or gradient with respect to the longitudinal axis LA.

In operation, as the inner container is inserted into the outer housing 200, the ramp portion of the protrusions 950A, 950B on the inner container 350 make contact with the corresponding inner wall of the outer container 200, which therefore flexes outwards a little. Eventually, when the inner container is fully inserted, as shown in Figure 9A, the steep sides of the protrusions 950A, 950B, which face in a direction away from the mouth end 35, abut against the steep side of the groove 905, which faces in the opposite direction (towards the mouth end). These two sides abut against one another at groove locations 905A, 905B to provide a latching action for latch mechanism 500, and thereby prevent withdrawal of the inner container 350 from the outer housing 200.

One particular advantage of the implementation shown in Figures 9A-9C is again there is no need to rotationally align the inner container 350 relative to the outer housing 200 about the longitudinal axis LA, since the inward groove 905 of the outer housing spans a rotational angle of 360 degrees. Accordingly, the groove 905 of the outer housing 200 will engage with the protrusions 950A, 950B of the inner container irrespective of the relative rotational angle of insertion between the outer housing 200 and the inner container 350. This therefore avoids the need to perform a rotational alignment between these two components prior to insertion of the inner container 350 into the outer housing 200, which can help to reduce manufacturing complexity (and hence costs).

Figure 10 illustrates another implementation of the cartridge 30 which again comprises an outer housing 200 and an inner container 350. In particular, Figure 10 is a horizontal cross-section through the cartridge 30 (including the longitudinal axis LA). The implementation of Figure 10 is generally the same as the implementation of Figures 9A-9C, in that has a circumferential groove 1005 is formed on the interior cylindrical wall of the outer housing 200, and this forms a latching mechanism 500 with two corresponding protrusions 1050A, 1050B on respective sides of the inner container 350.

The implementation of Figure 10 differs from the implementation of Figures 9A-9C as regards the positioning of the circumferential groove 1005 along the longitudinal axis LA, and hence the corresponding positioning of the protrusions 1050A, 1050B. In particular, the protrusions 1050A, 1050B are now located at the end of the inner container furthest from the mouth end 35 (analogous to tail fins). This positioning may provide certain advantages. For example, the flexing of the outer housing to accommodate the protrusions 1050A, 1050B as the inner container 350 is inserted into the outer housing 200 prior to engagement of the latching mechanism 500 occurs further away from the end wall 39 and mouth end 35 and nearer to the (opposite) open end of the outer housing 200. It will be appreciated that this open end will naturally have slightly increased flexibility.

Although various latching mechanisms 500 have been disclosed herein, it will be appreciated that these are presented by way of example, and many additional possibilities as to the shape, positioning, operation etc of the latching mechanism will be apparent to a person of ordinary skill in the art. Moreover, although the e-cigarette described herein comprises three detachable sections, namely the control unit, cartridge and vaporiser, it will be appreciated that other e-cigarettes may comprise a different number of sections.

In order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). Various embodiments may suitably comprise, consist of, or consist essentially of, various combinations of the disclosed elements, components, features, parts, steps, means, etc other than those specifically described herein.

## Claims

1. A cartridge (30) for use in a vapour provision system (10), wherein the cartridge includes an inner container (350) holding a reservoir of fluid to be vapourised, and an outer housing (200) having a mouthpiece (35) formed therein, wherein the outer housing (200) extends in a longitudinal direction along the outside of the inner container (350) for at least a substantial portion of the inner container (350), and wherein the inner container (350) and outer housing (200) are provided with a latch mechanism (500) to retain the inner container (350) within the outer housing (200), **characterized in that** the outer housing comprises a mechanical connector for longitudinally attaching the cartridge to a control unit of a vapour provision system.

2. The cartridge (30) of claim 1, wherein the latch mechanism (500) is operable independent of the relative rotational angle between the inner container (350) and the outer housing (200) with respect to the longitudinal axis.

3. The cartridge (30) of claim 1 or 2, wherein the latch mechanism (500) includes a first member formed on one of the outer housing (200) or the inner container (350), and a cooperating second member formed on the other of the outer housing (200) or the inner container (350), and wherein the first and second members abut one another to engage the latch mechanism (500).

4. The cartridge (30) of claim 3, wherein the first and second members are located at or near the end of the inner container (350) which is longitudinally opposite to the location of the mouthpiece (35).

5. The cartridge (30) of claim 3, wherein the inner container (350) includes a body holding said reservoir of fluid and a tab (358), wherein the tab (358) separates the body from the mouthpiece (35) to allow airflow through the mouthpiece (35), and wherein the first and second members are longitudinally located adjacent to said tab (358).

6. The cartridge (30) of any of claims 3 to 5, wherein the first member comprises a radial directed groove (905) in the inner surface of the outer housing (200) and the second member comprises at least one radially directed protrusion (950B) on the outer surface of the inner container (350).

7. The cartridge (30) of any of claims 3 to 5, wherein the first member comprises a radial directed protrusion (505) from the inner surface of the outer housing (200) and the second member comprises at least one radially directed groove (510) in the outer surface of the inner container (350).

8. The cartridge (30) of any of claims 3 to 5, wherein the first member comprises a radial directed protrusion (805) from the inner surface of the outer housing (200) and the second member comprises at least one radially directed protrusion (850) on the outer surface of the inner container (350).

9. The cartridge (30) of any of claims 3 to 8, wherein at least one of the first or second members extends circumferentially around an axis extending in said longitudinal direction.

10. The cartridge (30) of any of claims 3 to 9, wherein the first and second members are provided with ramp portions that slide past one another as the inner container (350) is inserted into the outer housing (200) prior to engagement of the latch.

11. The cartridge (30) of any preceding claim, wherein a channel (355) is provided between the inner surface of the outer housing (200) and the outer surface of the inner container (350) to allow vapour to flow longitudinal through the channel to the mouthpiece (35).

12. The cartridge (30) of claim 11, wherein the outer housing (200) has a substantially circular cross-section with respect to an axis extending in said longitudinal direction and the inner container (350) has a substantially D-shaped cross-section with respect to said longitudinal axis to provide said channel, optionally wherein the latch mechanism (500) is located within said channel, and is sized so as not to obstruct said channel.

13. The cartridge (30) of any preceding claim, wherein at least one of the outer housing (200) and the inner container (350) is sufficiently flexibly resilient to support operation of the latch mechanism (500).

14. The cartridge (30) of any preceding claim, wherein the cartridge includes a vaporiser (40), optionally wherein the mechanical connector further provides an electrical connection (21A) for receiving electrical power from the control unit (20) for operating the vaporiser.

15. A vapour provision system (10) including the cartridge (30) of any preceding claim.

## Patentansprüche

1. Patrone (30) zum Gebrauch in einem Dampfbereitstellungssystem (10), wobei die Patrone einen Innenbehälter (350), der einen Vorrat an zu verdampfender Flüssigkeit enthält, und ein Außengehäuse (200), das ein in demselben ausgebildetes Mundstück (35) aufweist, umfasst, wobei sich das Außengehäuse (200) in einer Längsrichtung entlang der Außenseite des Innenbehälters (350) über einen wenigstens wesentlichen Abschnitt des Innenbehälters (350) erstreckt und wobei der Innenbehälter (350) und das Außengehäuse (200) mit einem Verriegelungsmechanismus (500) versehen sind, um den Innenbehälter (350) in dem Außengehäuse (200) zu halten, **dadurch gekennzeichnet, dass** das Außengehäuse einen mechanischen Verbinder umfasst, um die Patrone in Längsrichtung an einer Steuereinheit eines Dampfbereitstellungssystems anzubringen.

2. Patrone (30) nach Anspruch 1, wobei der Verriegelungsmechanismus (500) unabhängig von dem relativen Drehwinkel zwischen dem Innenbehälter (350) und dem Außengehäuse (200) in Bezug auf die Längsachse betätigbar ist.

3. Patrone (30) nach Anspruch 1 oder 2, wobei der Verriegelungsmechanismus (500) ein erstes Element, das an dem einen von dem Außengehäuse (200) oder dem Innenbehälter (350) ausgebildet ist, und ein zusammenwirkendes zweites Element, das an dem anderen von dem Außengehäuse (200) oder dem Innenbehälter (350) ausgebildet ist, umfasst und wobei das erste und das zweite Element aneinander anliegen, um mit dem Verriegelungsmechanismus (500) einzugreifen.

4. Patrone (30) nach Anspruch 3, wobei sich das erste und das zweite Element an dem Ende des Innenbehälters (350) oder in der Nähe desselben befinden, das in Längsrichtung der Stelle des Mundstücks (35) gegenüberliegt.

5. Patrone (30) nach Anspruch 3, wobei der Innenbehälter (350) einen Körper, der den Vorrat an Flüssigkeit enthält, und eine Lasche (358) umfasst, wobei die Lasche (358) den Körper von dem Mundstück (35) trennt, um einen Luftstrom durch das Mundstück (35) zu ermöglichen, und wobei sich das erste und das zweite Element in Längsrichtung angrenzend an die Lasche (358) befinden.

6. Patrone (30) nach einem der Ansprüche 3 bis 5, wobei das erste Element eine radial ausgerichtete Rille (905) in der Innenfläche des Außengehäuses (200) umfasst und das zweite Element wenigstens einen radial ausgerichteten Vorsprung (950B) auf der Außenfläche des Innenbehälters (350) umfasst.

7. Patrone (30) nach einem der Ansprüche 3 bis 5, wobei das erste Element einen radial ausgerichteten Vorsprung (505) von der Innenfläche des Außengehäuses (200) umfasst und das zweite Element wenigstens eine radial ausgerichtete Rille (510) in der Außenfläche des Innenbehälters (350) umfasst.

8. Patrone (30) nach einem der Ansprüche 3 bis 5, wobei das erste Element einen radial ausgerichteten Vorsprung (805) von der Innenfläche des Außengehäuses (200) umfasst und das zweite Element wenigstens einen radial ausgerichteten Vorsprung (850) auf der Außenfläche des Innenbehälters (350) umfasst.

9. Patrone (30) nach einem der Ansprüche 3 bis 8, wobei sich das erste und/oder das zweite Element in Umfangsrichtung um eine Achse erstrecken bzw. erstreckt, die in der Längsrichtung verläuft.

10. Patrone (30) nach einem der Ansprüche 3 bis 9, wobei das erste und das zweite Element mit Rampenabschnitten versehen sind, die aneinander vorbeigleiten, während der Innenbehälter (350) in das Außengehäuse (200) vor dem Eingriff mit der Verriegelung eingesetzt wird.

11. Patrone (30) nach einem der vorhergehenden Ansprüche, wobei ein Kanal (355) zwischen der Innenfläche des Außengehäuses (200) und der Außenfläche des Innenbehälters (350) bereitgestellt ist, um zu ermöglichen, dass Dampf in Längsrichtung durch den Kanal zu dem Mundstück (35) strömt.

12. Patrone (30) nach Anspruch 11, wobei das Außengehäuse (200) in Bezug auf eine Achse, die in der Längsrichtung verläuft, einen im Wesentlichen kreisrunden Querschnitt aufweist und der Innenbehälter (350) in Bezug auf die Längsachse einen im Wesentlichen D-förmigen Querschnitt aufweist, um den Kanal bereitzustellen, wobei sich der Verriegelungsmechanismus (500) optional in dem Kanal befindet und so dimensioniert ist, dass er den Kanal nicht blockiert.

13. Patrone (30) nach einem der vorhergehenden Ansprüche, wobei das Außengehäuse (200) und/oder der Innenbehälter (350) ausreichend flexibel nachgiebig sind bzw. ist, um eine Betätigung des Verriegelungsmechanismus (500) zu unterstützen.

14. Patrone (30) nach einem der vorhergehenden Ansprüche, wobei die Patrone einen Verdampfer (40) umfasst, wobei der mechanische Verbinder ferner optional einen elektrischen Anschluss (21A) bereitstellt, um zur Betätigung des Verdampfers elektrischen Strom von der Steuereinheit (20) zu erhalten.

15. Dampfbereitstellungssystem (10), das die Patrone (30) nach einem der vorhergehenden Ansprüche umfasst.

## Revendications

1. Cartouche (30) à utiliser dans un système de distribution de vapeur (10), la cartouche comprenant un conteneur interne (350) retenant un réservoir de fluide à vaporiser, et un logement externe (200) possédant un embout (35) formé dessus, dans laquelle le logement externe (200) s'étend dans une direction longitudinale le long de l'extérieur du conteneur interne (350) sur au moins une partie substantielle du conteneur interne (350), et dans laquelle le conteneur interne (350) et le logement externe (200) sont dotés d'un mécanisme de verrouillage (500) destiné à retenir le conteneur interne (350) à l'intérieur du logement externe (200), **caractérisée en ce que** le logement externe comprend un connecteur mécanique destiné à fixer longitudinalement la cartouche à une unité de commande d'un système de distribution de vapeur.

2. Cartouche (30) selon la revendication 1, dans laquelle le mécanisme de verrouillage (500) peut fonctionner indépendamment de l'angle de rotation relatif entre le conteneur interne (350) et le logement externe (200) par rapport à l'axe longitudinal.

3. Cartouche (30) selon la revendication 1 ou 2, dans laquelle le mécanisme de verrouillage (500) inclut un premier élément formé sur le logement externe (200) ou le conteneur interne (350), et un second élément coopérant formé sur l'autre du logement externe (200) ou du conteneur interne (350), et dans laquelle les premier et second éléments viennent en butée l'un contre l'autre pour venir en prise avec le mécanisme de verrouillage (500).

4. Cartouche (30) selon la revendication 3, dans laquelle les premier et second éléments sont situés à ou proches de l'extrémité du conteneur interne (350) qui est longitudinalement opposée à l'emplacement de l'embout (35).

5. Cartouche (30) selon la revendication 3, dans laquelle le conteneur interne (350) comprend un corps retenant ledit réservoir de fluide et une languette (358), dans laquelle la languette (358) sépare le corps de l'embout (35) pour permettre la circulation d'air à travers l'embout (35), et dans laquelle les premier et second éléments sont situés longitudinalement adjacents à ladite languette (358).

6. Cartouche (30) selon l'une quelconque des revendications 3 à 5, dans laquelle le premier élément comprend une rainure orientée radialement (905) dans la surface intérieure du logement externe (200) et le second élément comprend au moins une saillie orientée radialement (950B) sur la surface extérieure du conteneur interne (350).

7. Cartouche (30) selon l'une quelconque des revendications 3 à 5, dans laquelle le premier élément comprend une saillie orientée radialement (505) depuis la surface intérieure du logement externe (200) et le second élément comprend au moins une rainure orientée radialement (510) dans la surface extérieure du conteneur interne (350).

8. Cartouche (30) selon l'une quelconque des revendications 3 à 5, dans laquelle le premier élément comprend une saillie orientée radialement (805) depuis la surface intérieure du logement externe (200) et le second élément comprend au moins une saillie orientée radialement (850) sur la surface extérieure du conteneur interne (350).

9. Cartouche (30) selon l'une quelconque des revendications 3 à 8, dans laquelle au moins l'un des premier et second éléments s'étend circonférentiellement autour d'un axe s'étendant dans ladite direction longitudinale.

10. Cartouche (30) selon l'une quelconque des revendications 3 à 9, dans laquelle les premier et second éléments sont dotés de parties en pente qui coulissent l'une derrière l'autre lorsque le conteneur interne (350) est inséré dans le logement externe (200) avant la prise du verrou.

11. Cartouche (30) selon l'une quelconque des revendications précédentes, dans laquelle un canal (355) est prévu entre la surface intérieure du logement externe (200) et la surface extérieure du conteneur interne (350) pour permettre à la vapeur de circuler longitudinalement à travers le canal jusqu'à l'embout (35) .

12. Cartouche (30) selon la revendication 11, dans laquelle le logement externe (200) présente une section transversale sensiblement circulaire par rapport à un axe s'étendant dans ladite direction longitudinale et le conteneur interne (350) présente une section transversale sensiblement en forme de D par rapport audit axe longitudinal pour produire ledit canal, facultativement dans laquelle le mécanisme de verrouillage (500) est situé à l'intérieur dudit canal et est dimensionné de façon à ne pas obstruer ledit canal.

13. Cartouche (30) selon l'une quelconque des revendications précédentes, dans laquelle au moins l'un du logement externe (200) et du conteneur interne (350) est suffisamment flexible élastiquement pour supporter le fonctionnement du mécanisme de verrouillage (500).

14. Cartouche (30) selon l'une quelconque des revendications précédentes, la cartouche incluant un vaporisateur (40), facultativement dans laquelle le connecteur mécanique fournit en outre une connexion électrique (21A) destinée à recevoir une alimentation électrique depuis l'unité de commande (20) pour faire fonctionner le vaporisateur.

15. Système de distribution de vapeur (10) incluant la cartouche (30) selon l'une quelconque des revendications précédentes.
